# EUROPEAN PATENT SPECIFICATION

(11) **EP 3 574 940 B1**
(45) Date of publication and mention of the grant of the patent: **21.10.2020**
(21) Application number: 19174695.7
(22) Date of filing: 15.05.2019
(51) Int. Cl.: A61M 5/14, A61M 5/162, A61J 1/20

(54) **DRIP CHAMBER AND SPIKE ASSEMBLY FOR MEDICAL LINES**
TROPFKAMMER UND SPIKE-ANORDNUNG FÜR MEDIZINISCHE LEITUNGEN
CHAMBRE DE GOUTTE-À-GOUTTE ET DISPOSITIF POURVU D'UNE AUGIULLE POUR LIGNES MÉDICALES

(30) Priority: 01.06.2018 IT 201800002661 U
(43) Date of publication of application: 04.12.2019
(73) Proprietor: Industrie Borla S.p.A., 10024 Moncalieri (Torino) (IT)
(72) Inventor: GUALA, Gianni, I-10133 Torino (IT)
(74) Representative: Buzzi, Franco

(56) References cited:
- DE-A1- 3 019 771
- DE-U1- 29 907 462
- US-A- 3 868 965
- US-A- 4 055 176
- US-A- 4 857 068
- US-A1- 2009 259 199

## Description

### Field of the invention

The present invention refers to the medical devices consisting of a drip chamber and spike assembly.

### State of the art

Devices thus made, typically used in infusion or transfusion medical lines, allow to supply - in a dosed manner - to a patient a medical liquid for example contained in a container through which the spike assembly is inserted. For instance, US3868965 and US4857068 disclose devices used for infusion of a medical liquid to a patient, wherein the device includes a drip chamber and a spike assembly.

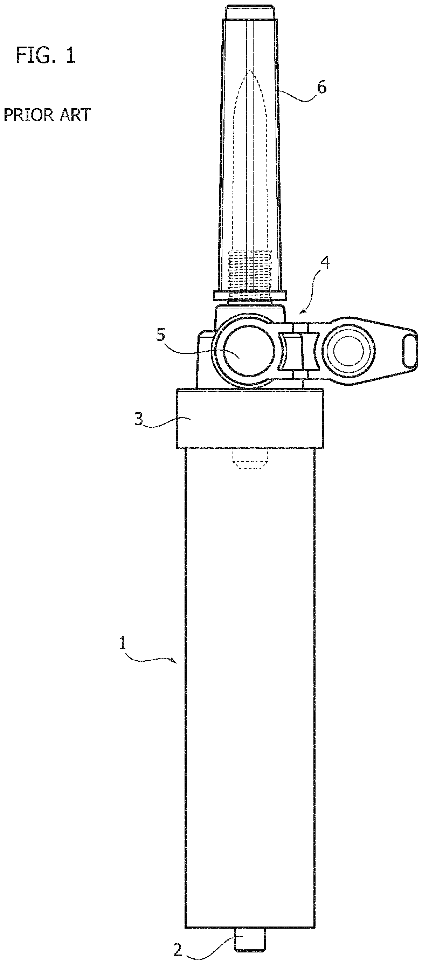

Figure 1 of the attached drawings schematically illustrates a drip chamber and spike assembly according to the prior art, corresponding to what is currently manufactured and sold by the Applicant. As illustrated in such figure the drip chamber consists in a generally cylindrical body 1, normally made of transparent plastic material, formed at one end with a tubular connector 2 for connection to a flexible duct and whose opposite end edge is fixed to a base part 3 of the spike assembly 4, normally provided with a filter with plug 5 and enclosed in a removable protection cap 6.

The base part 3 of the known spike assembly 4 comprises an outer wall visible in the figure and an inner wall not visible, which delimit between them an annular interspace in which the end edge of the body 1 of the drip chamber is engaged and fixed for example by means of gluing. The outer wall of the base part 3 of the spike assembly 4 also serves as a grasping element for handling the drip chamber 1.

This known conformation requires, as regards the coupling methods between the drip chamber and the spike assembly, a given amount of material which, multiplied by the considerable number of articles manufactured (in the order of a few millions per year), entails significant overall costs. Furthermore, the practicality of the handling carried out in use through the part 3 of the spike assembly 4 is low.

### Summary of the invention

The object of the present invention is to considerably reduce the amount of the material required for manufacturing the drip chamber and spike assembly, so as to equally significantly reduce the manufacturing costs and thus the price, while simultaneously improving the handling comfort and practicability thereof for the operators.

According to the invention, this object is attained thanks to the fact that the base part of the spike assembly to which the drip chamber is fixed solely consists of a hollow tang engaged in the aforementioned end edge of the body of the drip chamber, and such end edge of the drip chamber is formed externally with a crown of integral reliefs defining a grasping element.

In this manner, the manufacturing and the assembly of the drip chamber and spike assembly are simplified, and the grasping is basically transferred from the spike assembly to the drip chamber.

The body of the drip chamber conveniently consist of a soft plastic material, which allows to further improve the handling by means of the relative grasping element.

### Brief description of the drawings

The invention will now be described in detail, purely by way of non-limiting example, with reference to the attached drawings, wherein:
- figure 1 is an elevational schematic view of a drip chamber and spike assembly according to the prior art described previously,
- figure 2 is a view similar to figure 1 which represents a first embodiment of the drip chamber and spike assembly according to the invention, and
- figure 3 is a view similar to figure 2 which represents a variant of the drip chamber and spike assembly not part of the invention, and

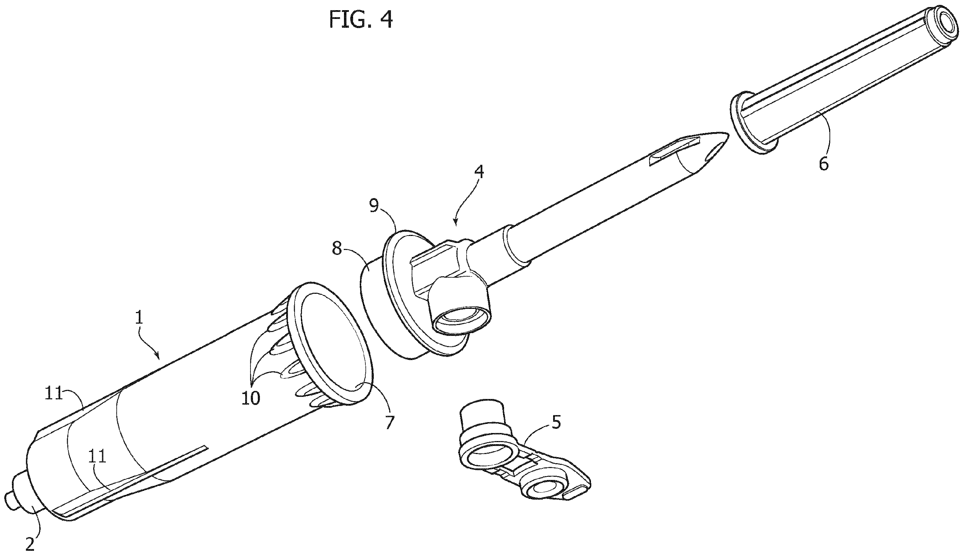

- figure 4 is an exploded perspective view of the drip chamber and spike assembly of figure 2.

### Detailed description of the invention

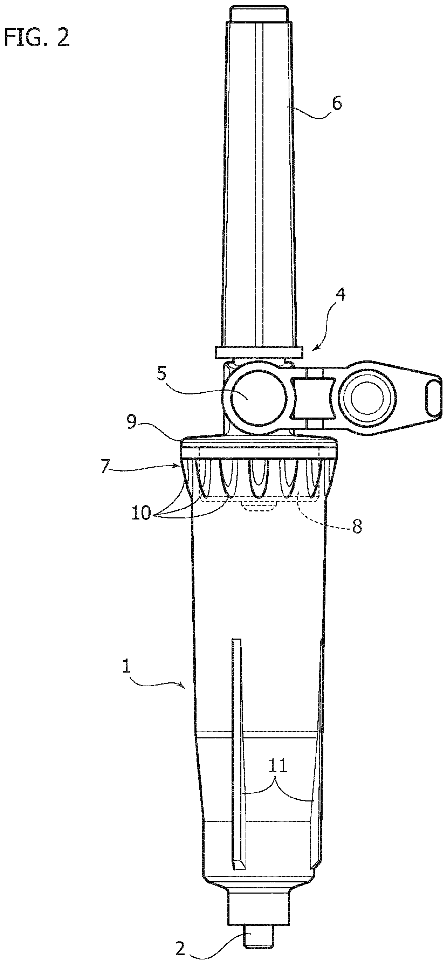

Initially with reference to figure 2, in which parts identical or similar to those described previously with reference to figure 1 are indicated using the same reference numbers, the end edge of the body 1 of the drip chamber, indicated with 7, is fitted and thus for example fixed by gluing on a simple hollow tang 8 with complementary shape located at the base of the spike assembly 4 and projecting beyond a thin annular stop flange 9. As observable by comparison with figure 1, with this arrangement the amount of material required to manufacture the spike assembly 4 is considerably lesser, to the advantage of lower manufacturing cost of the entire article. The methods of assembly between the body 1 of the drip chamber and the spike assembly 4 are simplified too.

The end edge 7 of the body 1 of the drip chamber externally consists of a crown of axial integral reliefs 10 which define a grasping element for the practical and comfortable handling of the drip chamber. In this case, the body 1 of the drip chamber is conveniently made of a soft plastic material, typically PVC, and it externally consists of thin stiffening axial ribs 11. The spike assembly 4 is normally made of rigid plastic material.

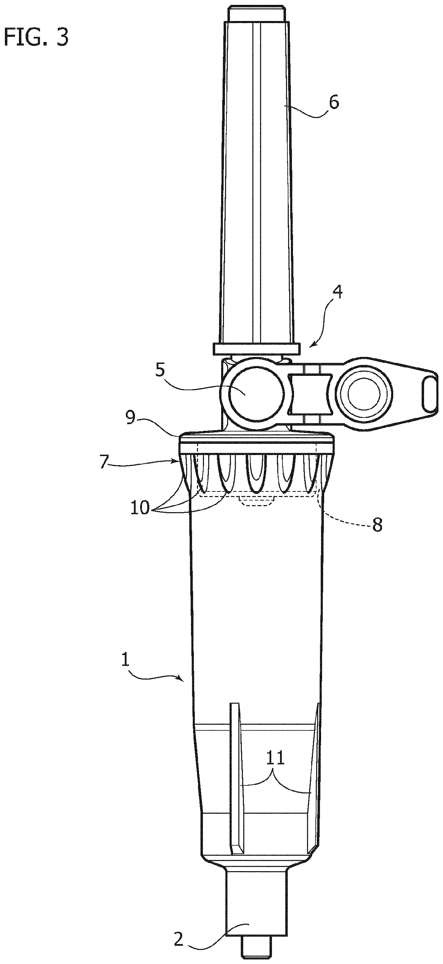

The variant represented in figure 3, not part of the invention, is similar to the embodiment of figure 2 with the sole difference lying in that the material of the body 1 of the drip chamber is relatively rigid, and in this case the stiffening ribs 11 can be less accentuated, or even absent.

Obviously, the construction details and the embodiments may widely vary with respect to what has been described and illustrated, without departing from the scope of protection of the present invention as defined in the claim that follows.

## Claims

1. Drip chamber and spike assembly for medical lines, including a generally cylindrical body (1) having an end edge (7) fixed to a base part(3) of the spike assembly (4), wherein said body (1) of the drip chamber is made of a soft plastic material and said base part solely consists of a hollow tang (8) engaged in said end edge (7) of the body (1) of the drip chamber, **characterised by** said end edge (7) of the body (1) of the drip chamber externally consisting of a crown of integral reliefs (10) defining a grasping element.

## Patentansprüche

1. Tropfkammer und Spike-Anordnung für medizinische Leitung,
die einen allgemein zylinderförmigen Körper (1) mit einer Endkante (7), die an einem Unterteil (3) der Spike-Anordnung (4) befestigt ist, enthält,
wobei der Körper (1) der Tropfkammer aus einem weichen Kunststoffmaterial gefertigt ist und das Unterteil nur aus einem Hohlzapfen (8) besteht, der in die Endkante (1) des Körpers (1) der Tropfkammer eingreift,
**dadurch gekennzeichnet, dass**
die Endkante (7) des Körpers (1) der Tropfkammer extern aus einem Kranz aus integralen Erhebungen (10), die ein Greifelement definieren, besteht.

## Revendications

1. Chambre à gouttes et assemblage de pointe pour lignes médicales, incluant un corps généralement cylindrique (1) ayant un bord d'extrémité (7) fixé à une partie de base (3) de l'assemblage de pointe (4), où ledit corps (1) de la chambre à gouttes est fait d'un matériau plastique souple et ladite partie de base consiste uniquement en un tenon creux (8) engagé dans ledit bord d'extrémité (7) du corps (1) de la chambre à gouttes, **caractérisée en ce que** ledit bord d'extrémité (7) du corps (1) de la chambre à gouttes consiste extérieurement en une couronne de reliefs intégrés (10) définissant un élément de préhension.
